# EUROPEAN PATENT APPLICATION

(11) **EP 0 604 653 A1**
(43) Date of publication of application: **06.07.1994**
(21) Application number: 92920108.5
(22) Date of filing: 21.09.1992
(51) Int. Cl.: C07C 59/90, A61K 31/19, C07C 69/738, A61K 31/215, C08B 37/16

(54) **COMPLEX OF COMPOUND HAVING CHALCONE SKELETON**

(30) Priority: 21.09.1991 JP 242127/91
(71) Applicant: TAISHO PHARMACEUTICAL CO. LTD, Tokyo 171 (JP)
(72) Inventor: ITO, Shusei, Toshima-ku Tokyo 171 (JP); ITAI, Shigeru, Toshima-ku Tokyo 171 (JP); UMEKI, Nobuo, Toshima-ku Tokyo 171 (JP)
(74) Representative: Kraus, Walter, Dr.
(86) International application number: JP9201199
(87) International publication number: WO9306075

(57) **Abstract**

A solid preparation exhibiting a good absorbability of 4-t-butyl-2'-carboxymethoxy-4'-(3-methyl-2-butenyloxy)chalcone and 2'-carboxymethoxy-4,4'-bis(3-methyl-2-butenyloxy)chalcone and available for treating gastric ulcer and gastritis as a protective factor. A complex having an improved bioavailability and an excellent antiulcer activity is provided by forming a clathrate complex between the chalcone and cyclodextrin.

## Description

### FIELD OF THE INVENTION

This invention relates to a complex of a compound having a chalcone skeleton. More particularly, it relates to a complex of a compound having a chalcone skeleton, which is effective as a protective factor against gastric ulcer, with a cyclodextrin.

### BACKGROUND OF THE INVENTION

A compound having a chalcone skeleton is known to be effective as a protective factor against gastric ulcer. In particular, 4-t-butyl-2'-carboxymethoxy-4'-(3-methyl-2-butenyloxy)chalcone (hereinafter referred to as SU-740) disclosed in EP412803 and 2'-carboxymethoxy-4,4'-bis(3-methyl-2-butenyloxy)chalcone disclosed in JP-A-54-32634 (the term "JP-A" as used herein means "unexamined published Japanese Patent Application"), which is known as sofalcone in general name, show high effects in the factor.

Since these compounds having a chalcone skeleton have low bioavailability, fine grinds, solid dispersions, soft capsules of a solution in an oil base, etc. have hitherto been reported in order to improve their bioavailability.

### DISCLOSURE OF THE INVENTION

In order to solve the above-mentioned problem, the present inventors have conducted extensive investigations. As a result, it was found that a complex of a compound having a chalcone skeleton with an improved bioavailability can be obtained by preparing a clathrate complex from a compound having a chalcone skeleton and a cyclodextrin. The present invention has been completed based on this finding.

Namely, the present invention relates to a compound having a chalcone skeleton-cyclodextrin complex obtained from a compound having a chalcone skeleton and a cyclodextrin, in which the chalcone compound is SU-740 or sofalcone.

The compound having a chalcone skeleton-cyclodextrin complex according to the present invention can be prepared, for example, by a process comprising adding a small amount of water to a mixture of a compound having a chalcone skeleton and a cyclodextrin and kneading the mixture in a mortar, followed by drying. Further, it can also be prepared by a process comprising adding an ethyl ether solution of a chalcone compound to a saturated cyclodextrin solution, collecting the precipitate thus formed by filtration, and drying the precipitate. Furthermore, it can be prepared by a process comprising dissolving a compound having a chalcone skeleton and a cyclodextrin in water under heating with stirring and then spray-drying or lyophilizing the solution.

The compound having a chalcone skeleton which can be used in the present invention includes SU-740 and sofalcone. The cyclodextrin which can be used in the present invention includes α-cyclodextrin, β-cyclodextrin, hydroxypropyl-β-cyclodextrin, and γ-cyclodextrin, with β-cyclodextrin and hydroxypropyl-β-cyclodextrin being preferred. The amount of cyclodextrin to be used is from 0.1 to 3.0 mols per mol of the compound having a chalcone skeleton. A preferred amount of the cyclodextrin is from 1.0 to 3.0 mols per mol of the compound having a chalcone skeleton and a particularly preferred amount is 1.0 to 2.0 mols per mol of the compound having a chalcone skeleton.

The above-mentioned preparations according to the present invention may contain pharmaceutically acceptable additives, such as those essential to formulations, e.g., vehicles, disintegrators, binders and lubricants; and those used in trace amounts for adding commercial values, e.g., colorants and flavors.

Usable vehicles and disintegrators include corn starch, lactose, crystalline cellulose, and calcium carboxymethyl cellulose. Usable binders include gelatin, methyl cellulose, and hydroxypropyl cellulose. Usable lubricants include magnesium stearate, talc, and hydrogenated oil. Usable colorants and flavors include tar-based pigments, and natural or synthetic flavors. The pharmaceutically acceptable additives to be used are not particularly limited, and any of those generally employed in solid preparations can be used in the present invention.

A complex of SU-740 according to the present invention is usually administered orally 2 to 4 times a day at a single dose of 200 mg (corresponding to about 30 mg of SU-740) for adults.

### BRIEF DESCRIPTION OF DRAWINGS

FIGs. 1 and 2 show the results of measurement with a differential scanning calorimeter for sofalcone and its complex prepared in Example 1 and SU-740 and its complex prepared in Example 2, respectively.

FIGs. 3 and 4 show the powder X-ray diffraction pattern of sofalcone and its complex prepared in Example 1 and SU-740 and its complex prepared in Example 2, respectively.

FIG. 5 shows the results of a test of absorbability of the complex prepared in Example 2, a mere mixture of equal amounts of SU-740 and a cyclodextrin, and SU-740 alone.

FIG. 6 shows the results of a test of gastric mucous lesion inhibitory effects of the complex prepared in Example 2, β-cyclodextrin alone, and SU-740 alone.

### PREFERRED EMBODIMENTS OF THE INVENTION

The process for preparing the complex of the present invention and the effects of the complex will be described in detail with reference to Examples and Test Examples.

### EXAMPLE 1

To a mixture of 45 g (0.1 mol) of sofalcone and 227 g (0.2 mol) of β-cyclodextrin was added 10 mℓ of purified water, and the mixture was kneaded in a mortar for 1 hour and dried under reduced pressure at 50°C for 5 hours to obtain a sofalcone-β-dextrin complex.

### EXAMPLE 2

In 0.02% aqueous ammonia was dissolved 113 g (0.1 mol) of β-cyclodextrin, and 42 g (0.1 mol) of SU-740 was dissolved therein. The solution was spray-dried at 120°C to obtain an SU-740-β-cyclodextrin complex.

### EXAMPLE 3

In 0.02% aqueous ammonia was dissolved 113 g (0.1 mol) of β-cyclodextrin, and 42 g (0.1 mol) of SU-740 was dissolved therein. The solution was lyophilized to obtain an SU-740-β-cyclodextrin complex.

### TEST EXAMPLE 1

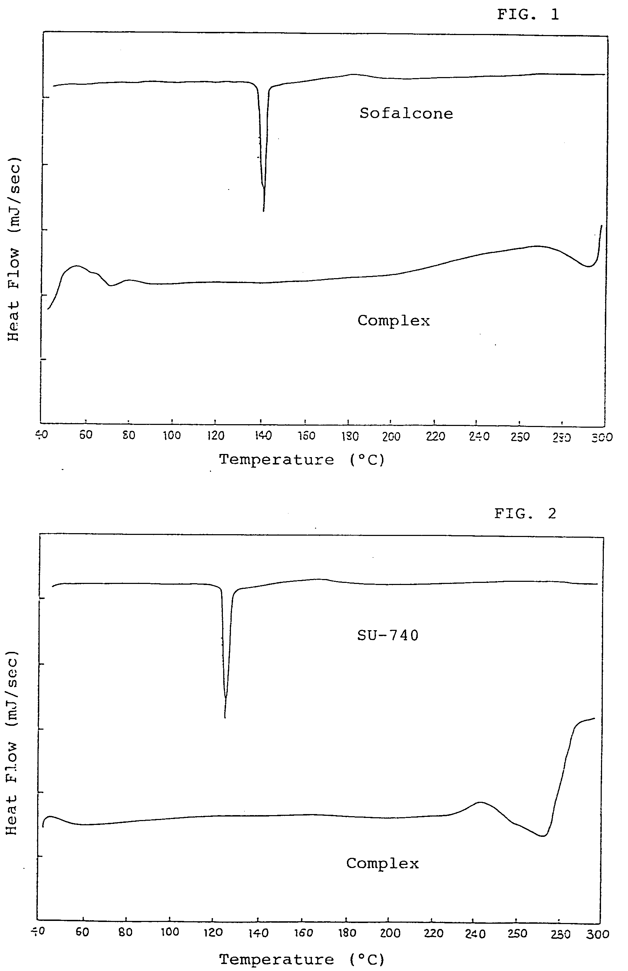

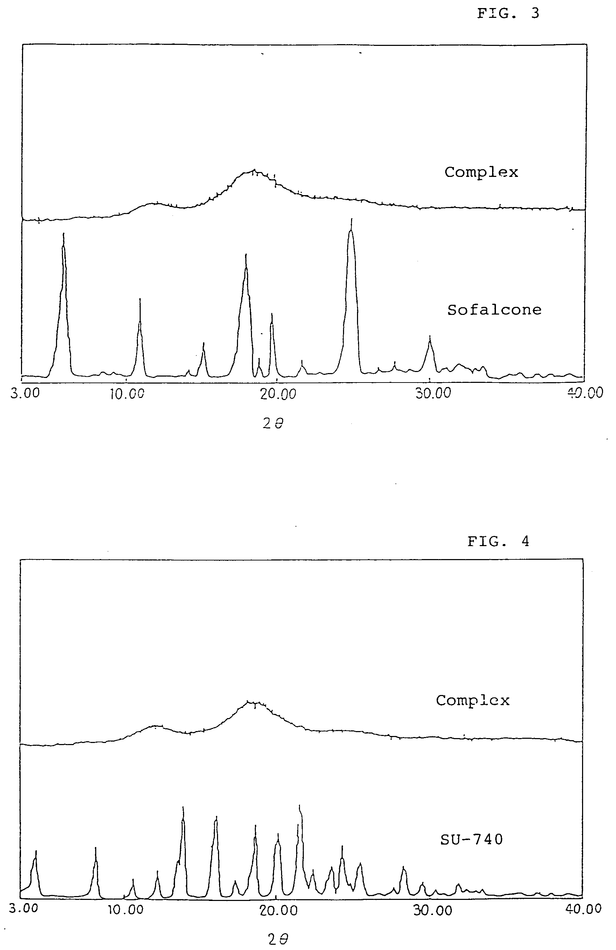

The complexes obtained in Examples 1 and 2 were analyzed by thermoanalysis and powder X-ray diffractometry to obtain the results shown in FIGs. 1 to 4. In each Figure, the peak assigned to the compound having a chalcone skeleton disappeared in the curve of the corresponding complex, revealing that the compound having a chalcone skeleton had been made amorphous.

### TEST EXAMPLE 2

A methanol extract and an acetonitrile extract of each of the complex prepared in Example 2 and a mere mixture of equal contents of SU-740 and a cyclodextrin were determined by liquid chromatography under the following conditions. As a result, the amount of the compound having a chalcone skeleton detected in the methanol or acetonitrile extract of the mere mixture agreed with the theoretical amount. On the other hand, while the content of the chalcone compound detected in the methanol extract of the complex agreed with the theoretical content, substantially no chalcone compound was detected from the acetonitrile extract of the complex. It was confirmed from these results that each of the raw materials and the complex differ in their behavior.

| Conditions of Liquid Chromatography: | |
|---|---|
| Column Size: | 4 φ x 150 mm |
| Packing: | TSK gel, ODS-80TM, 10 µm |
| Column Temp.: | 50°C |
| Mobile Phase: | acetonitrile/water/phosphoric acid = 700/300/1 |
| Detection Wave Length: | 230 nm |

### TEST EXAMPLE 3

### Tests of Absorbability on Complex obtained in Example 2, Mere Mixture of Equal Amounts of SU-740 and Cyclodextrin, and SU-740 Alone:

The test was conducted by a cross-over testing method according to a Latin square method using 15 beagles divided into three groups each consisting of 5 test animals. Each sample was put in a capsule and orally given to the animal so as to make a dose of SU-740 become 100 mg/kg.

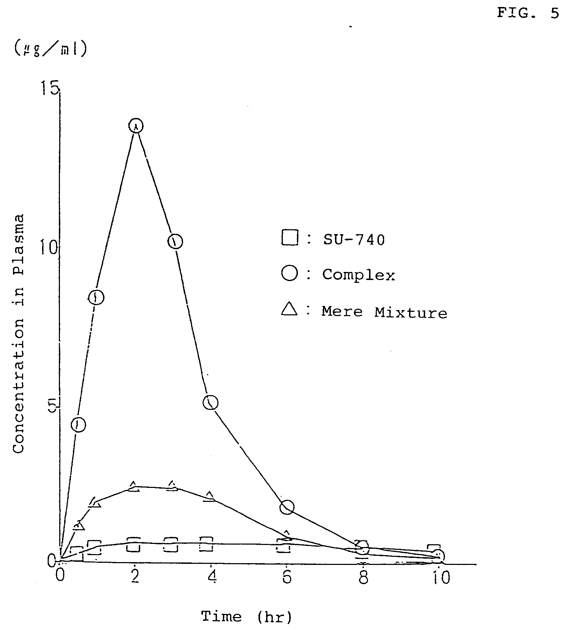

The results obtained are shown in FIG. 5. In this test example, it was confirmed that SU-740 when administered alone was scarcely absorbed, whereas the mixture with a cyclodextrin showed slightly improved absorbability, and the complex with a cyclodextrin exhibited remarkable improvement of absorbability.

### TEST EXAMPLE 4

### Effects of Complex obtained in Example 2, β-Cyclodextrin Alone and SU-740 Alone on Hydrochloric Acid-Induced Lesion:

Seventy-two rats divided into 9 groups each consisting of 8 animals were used. A 0.2 mℓ/100 g of the sample dispersed in a 5 % gum arabic suspension was intraduodenally administered to a rat so as to make a dose of SU-740 become 10 mg, 35 mg or 60 mg per kg-body weight. β-Cyclodextrin was similarly administered at a dose corresponding to the SU-740 content in the complex obtained in Example 2. On hour later, 1 mℓ of 0.6 N hydrochloric acid was administered, and 1 hour thereafter the abdomen was opened, and the length of the ulcerations was measured to evaluate the inhibitory effect on mucosal lesions.

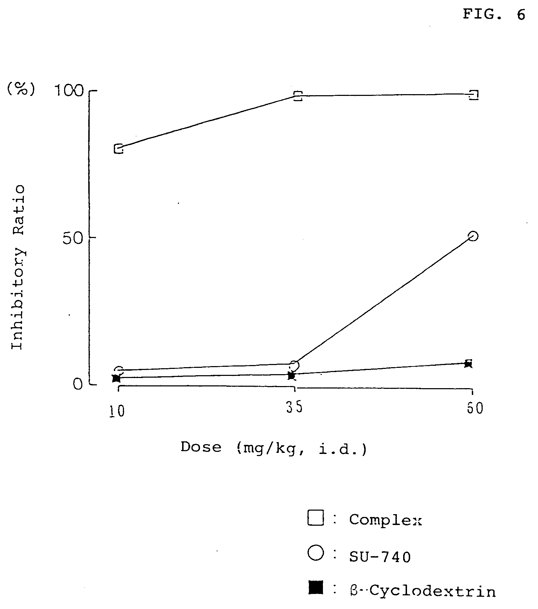

The results obtained are shown in FIG. 6. β-Cyclodextrin alone revealed little effect. On comparing the complex and the compound having a chalcone skeleton, the former exhibited a significantly higher inhibitory effect.

### TEST EXAMPLE 5

### Effects of Complex obtained in Example 2 and SU-740 on Histamine-Induced Ulceration:

The test was carried out in accordance with the method described in M. Muramatsu et al., Arzeneim. Forsch., Vol. 40(I)1, p. 49 (1990). The sample dispersed in a 1 % gum arabic suspension was intraduodenally administrated to rats having been deprived of food for 24 hours so as to make a dose of SU-740 become 31.4 mg/kg. Three hours later, 300 mg/kg of histamine was intraperitoneally administered, and 4 hours thereafter the abdomen was opened, and the area of lesions was measured to evaluate the inhibitory effect on mucosal lesions.

The results obtained are shown in Table 1.

**TABLE 1**

| Sample | Dose (mg/kg, i.d.) | Number of Animals | Inhibitory Ratio (%) |
|---|---|---|---|
| Control | - | 7 | - |
| SU-740 | 31.4 | 6 | 66.7 |
| Complex | 200.0 (31.4) | 7 | 91.7** |

| | | | |
|---|---|---|---|
| **: P<0.01 | | | |

The value in parentheses is the SU-740 content.

### TEST EXAMPLE 6

### Effects of Complex obtained in Example 2 and SU-740 on Prostaglandin (PG) Metabolic Enzyme Activity:

The test was carried out in accordance with the method described in M. Muramatsu et al., Biochemical Pharmacology, Vol. 33(16), p. 2629 (1984). The sample dispersed in a 1 % gum arabic suspension was intraduodenally administered to rats having been deprived of food for 24 hours so as to make a dose of SU-740 become 31.4 mg/kg. Two hours later, the gastric mucosa was excised, and a PG metabolic enzyme was extracted therefrom and analyzed with a spectrophotometer. The effect was examined taking decomposition of PGE₁ as an index.

The results obtained are shown in Table 2.

**TABLE 2**

| Sample | Dose (mg/kg, i.d.) | Number of Animals | PG Metabolic Enzyme Activity (nmol/mg protein/min) | % of Control |
|---|---|---|---|---|
| Control | - | 6 | 0.75±0.02 | 100.0 |
| SU-740 | 31.4 | 6 | 0.61±0.05 | 81.3 |
| Complex | 200.0 (31.4) | 6 | 0.29±0.01* | 38.7 |

| | | | | |
|---|---|---|---|---|
| *: P<0.05 | | | | |

The value in parentheses is the SU-740 content.

### TEST EXAMPLE 7

### Effects of Complex obtained in Example 2 and SU-740 on PGE₂ Content in Rat Gastric Mucosa:

The test was carried out in accordance with the method described in M. Muramatsu et al., Life Science, Vol. 41, p. 315 (1987). The sample dispersed in a 1 % gum arabic suspension was intraduodenally administered to rats having been deprived of food for 24 hours so as to make a dose of SU -740 become 31.4 mg/kg. After 24 hours from the administration, the gastric mucosa was excised under anesthesia with ether and 2 mℓ of a 20 µM anhydrous solution of Indomethacin was added thereto, followed by homogenization. After allowing to stand for 1 hour, the homogenate was subjected to centrifugation at 3000 rpm for 10 minutes and the supernatant liquor (PG mixture) was lyophilized. The resulting PG mixture was suspended in distilled water, and extracted with methanol by means of a column (vac elute). Then the extract was lyophilized, and subjected to measurement by means of a radioimmunoassay kit.

The results obtained are shown in Table 3.

**TABLE 3**

| Sample | Dose (mg/kg, i.d.) | Number of Animals | PGE₂ Content (pg/g wet weight) | % of Control |
|---|---|---|---|---|
| Control | - | 6 | 78.0±9.8 | 100.0 |
| SU-740 | 31.4 | 6 | 309.1±57.0** | 396.0 |
| Complex | 200.0 (31.4) | 6 | 685.9±116.3** | 879.4 |

| | | | | |
|---|---|---|---|---|
| *: P<0.01 | | | | |

The value in parentheses is the SU-740 content.

### TEST EXAMPLE 8

### Effects of Complex obtained in Example 2 and SU-740 on Gastric Acid Secretion in Conscious Dog with Chronic Fistula:

The test was carried out in accordance with the method described in Gastroenterology, Vol. 85, p. 900 (1983). A dog to which a fistula had been chronically fitted was deprived of food for 20 hours. The sample dispersed in a 1 % gum arabic suspension was orally given to the dog in conscious state so as to make a dose of SU-740 become 31.4 mg/kg. One hour later, 300 mg/kg of 2-deoxy-D-glucose was intravenously injected. Two hours from the injection, the gastric acid secretion was measured.

The results obtained are shown in Table 4.

**TABLE 4**

| Sample | Dose (mg/kg, p.o.) | Number of Animals | Gastric Acid Secretion (mEq/1 hr) | Inhibitory Ratio (%) |
|---|---|---|---|---|
| Control | - | 6 | 15.3±2.3 | - |
| SU-740 | 200.0 | 6 | 12.1±2.1 | 20.9 |
| Complex | 200.0 (31.4) | 6 | 5.6±1.1* | 63.4 |

| | | | | |
|---|---|---|---|---|
| *: P<0.05 | | | | |

The value in parentheses is the SU-740 content.

### Industrial Utility

The compound having a chalcone skeleton-cyclodextrin complex has markedly enhanced inhibitory effects on ulceration as well as improved solubility and absorbability. The complex can be formulated into preparations for oral administration, such as granules, tablets and capsules, in a usual manner for drug preparation.

## Claims

1. A complex obtained from a compound having a chalcone skeleton and a cyclodextrin.

## Amended claims

### Amended claims under Art. 19.1 PCT

(Amended) A cyclodextrin clathrate complex of 4-t-butyl-2'-carboxymethoxy-4'-(3-methyl-2-butenyloxy)chalcone.

(Added) A cyclodextrin clathrate complex of 2'-carboxymethoxy-4,4'-bis(3-methyl-2-butenyloxy)chalcone.
